# EUROPEAN PATENT APPLICATION

(11) **EP 4 446 416 A2**
(43) Date of publication of application: **16.10.2024**
(21) Application number: 22902538.2
(22) Date of filing: 06.12.2022
(51) Int. Cl.: C12N 15/62

(54) **CHIMERIC ENZYME AND METHOD FOR PRODUCING TERMINAL ALKENES**

(30) Priority: 06.12.2021 BR 102021024672
(71) Applicant: Centro Nacional de Pesquisa em Energia e Materiais - CNPEM, 13083-100 Campinas - SP (BR)
(72) Inventor: ZANPHORLIN MURAKAMI, Leticia Maria, 13083100 Campinas (BR); CARDOSO GENEROSO, Wesley, 13083100 Campinas (BR); FELIX PERSINOTI, Gabriela, 13083100 Campinas (BR)
(74) Representative: Studio Torta S.p.A.
(86) International application number: PCT/BR2022/050482
(87) International publication number: WO 2023/102629

(57) **Abstract**

The present invention refers to a chimeric enzyme constructed for the production of terminal alkenes from fatty acids, so that the cofactor necessary for the reaction is produced in situ from industrial waste, favoring the circular economy. Furthermore, the present invention deals with the process of producing terminal alkenes using said chimeric enzyme. This invention belongs to the field of industrial biotechnology and finds application in the biofuels and renewable chemicals industry.

## Description

### TECHNICAL FIELD

The present invention concerns a chimeric enzyme constructed for the production of terminal alkenes from fatty acids, as well as the process for the production of said alkenes using said enzyme. The present invention belongs to the field of industrial biotechnology and has application in the biofuels and renewable chemicals industry.

### BACKGROUND AND PRIOR ART

Terminal alkenes, or alpha olefins, are chemical compounds that can be synthesized from fatty acids of renewable origin. These molecules make up a relevant platform for the industrial production of polymers, fertilizers, lubricants, surfactants, biofuels and other compounds currently obtained via petrochemical routes, and the market size for them is projected at USD 13,464 million for 2022.

Given the relevance of these compounds for the chemical and biofuels industry, efforts to develop processes for producing terminal alkenes have been employed. Among the currently existing technologies for producing these molecules from fatty acids, the chemical route is one of the possibilities, but this is dependent on high temperature, high pressure and catalysis by heavy metals, which are harmful to the environment. Alternatively, the conversion of fatty acids into terminal alkenes can be carried out via a biotechnological route, using milder and more sustainable conditions. This route becomes especially interesting given the discussion about the need for responsible economic development, associated with environmental preservation. It is hoped that processes carried out in an environmentally sustainable way, with the use of materials and inputs from renewable sources or that make use of waste from other processes, become increasingly present on the world stage, contributing to the realization of the concept of Circular Economy.

Thus, within the scope of the biotechnological route for the production of terminal alkenes, scientific literature reports that the decarboxylase/peroxygenase enzyme called OleTJE, belonging to family 152 of the P450 superclass, has a unique property of removing oxygen from long-chain fatty acids. (C12-C20), generating 1-alkene as product. However, it is a very unstable enzyme, which is difficult to produce and requires high concentrations of salts (NaCl or KCl) for the decarboxylation reaction, which poses bottlenecks for its industrial use.

As well as OleTJE, there are enzymes from microorganisms such as *Kokuria rhizophila, Corynebacterium efficiens, Bacillus subtilis, Jeotgalicoccus sp., Staphylococcus massiliensis, Alicyclobacillus acidocaldarius* and other species, also with reported ability to convert fatty acids to terminal alkenes. However, a point to take into consideration is that, in general, the enzymatic route of fatty acids decarboxylation is dependent on hydrogen peroxide as a cofactor. The addition of peroxides to the reaction medium on an industrial scale imposes a cost and the need for an automatic injection mechanism at predetermined times.

A possible way to overcome the need to add hydrogen peroxide throughout the process is to generate it *in situ* for consumption during the conversion reaction. In this sense, CN107201356 (QINGDAO INSTITUTE OF BIOENERGY & BIOPROCESS TECHNOLOGY CAS BOEING INVESTMENT) presents, as a solution, the addition of a ferredoxin-ferredoxin reductase system to support the activity of the OleTJE enzyme. Similarly, CN112111477 (YANGZHOU UNIVERSITY) proposes the use of a glucose-glucose oxidase system for the generation of hydrogen peroxide, in a mild and controlled manner, to support an engineered enzyme (P450BS) in the conversion of fatty acids to alkenes. A similar proposal can also be found in EP1940348 (DUPONT NUTRITION BIOSCIENCES), which presents a composition comprising a system of coupled enzymes in which oxidoreductases generate hydrogen peroxide from sugars in the reaction medium for consumption by a decarboxylase enzyme.

It should be noted that, despite providing an alternative to the direct and controlled addition of hydrogen peroxide to the process, the solutions existing in the prior art impose an increase in the number of reagents necessary to carry out the conversion and require control so that both enzymes (peroxide generator and decarboxylase) are active under the reaction conditions. It is always interesting and preferable for a process to have few steps, ease of control and operation. Another point to note is that the coupling of an *in situ* peroxide generation system requires its own substrate for this reaction to take place. As described, the solutions proposed in the prior art employ ferredoxin or glucose/sugars as substrates, but one possibility of making the *in situ* peroxide generation process even more sustainable, adhering to the concept of circular economy, would be to carry out this reaction from waste from other processes.

Within this context, an extremely relevant waste in the current scenario is glycerol. In the production of biofuels alone, it is estimated that for every 1000 kilos of product such as bioethanol and biodiesel, 100 kilos of glycerol are generated. It is important to highlight that other important industries, such as oleochemistry, for example, also generate glycerol as a process residue. The volume of glycerol currently generated is greater than the capacity that the market can absorb. As a result, this waste has a low added value and, as an aggravating factor, the unused glycerol is destined for burning: in addition to the environmental problems inherent to the burning of compounds in general, the partial burning of glycerol generates acrolein, a molecule with carcinogenic potential. The conversion of glycerol through biotechnological processes into products with higher added value is a relevant alternative for reducing these problems.

Given this information, there are gaps to be filled and improved, aiming to make the biotechnological route of converting fatty acids to terminal alkenes even more practical and industrially attractive.

### SUMMARY OF THE INVENTION

As a way of solving the bottlenecks presented, the present invention is, in a first aspect, a chimeric enzyme constructed for the production of terminal alkenes from fatty acids. The chimeric enzyme of the present invention is the combination of a decarboxylase enzyme, which converts fatty acids to terminal alkenes, with a peroxide-generating enzyme, a cofactor of the reaction, by means of a linker sequence. The main advantage of the chimeric enzyme of the present invention is the fact that it has both functions necessary for the reaction in a single product, bringing economy and ease to the process. Furthermore, the enzyme of the present invention is capable of producing hydrogen peroxide *in situ* from glycerol, which favors a more sustainable industry, within the concept of circular economy, by allocating part of what would be disintegrated by burning to an application with greater added value. Furthermore, the chimeric enzyme of the present invention does not require salt-saturated media to carry out the conversion of fatty acids to alkenes.

It is worth noting that not every enzyme with any similarity to the one proposed in this invention will be capable of application in an industrial process as it does not have stability in the soluble form, a fundamental characteristic presented by the chimeric enzyme of the present invention. Furthermore, the joining of two or more sequences can result in a non-functional product or one that performs below expectations.

In this sense, given the aforementioned, the present invention is, in a second aspect, a process for producing alkenes from fatty acids using the chimeric enzyme referred to in this invention. As an advantage, the process of the present invention exhibits a similar performance to that of the conventional process, which involves the isolated decarboxylase enzyme and the direct addition of hydrogen peroxide to the medium, starting from low concentrations of the enzyme of the present invention, which reinforces that the sequences claimed below are functional and present an advance in relation to the prior art. Furthermore, the process provides an alternative destination to burning glycerol, using it to produce compounds with high added value and reducing the environmental impacts caused by the current management of this waste.

### BRIEF DESCRIPTION OF THE DRAWINGS

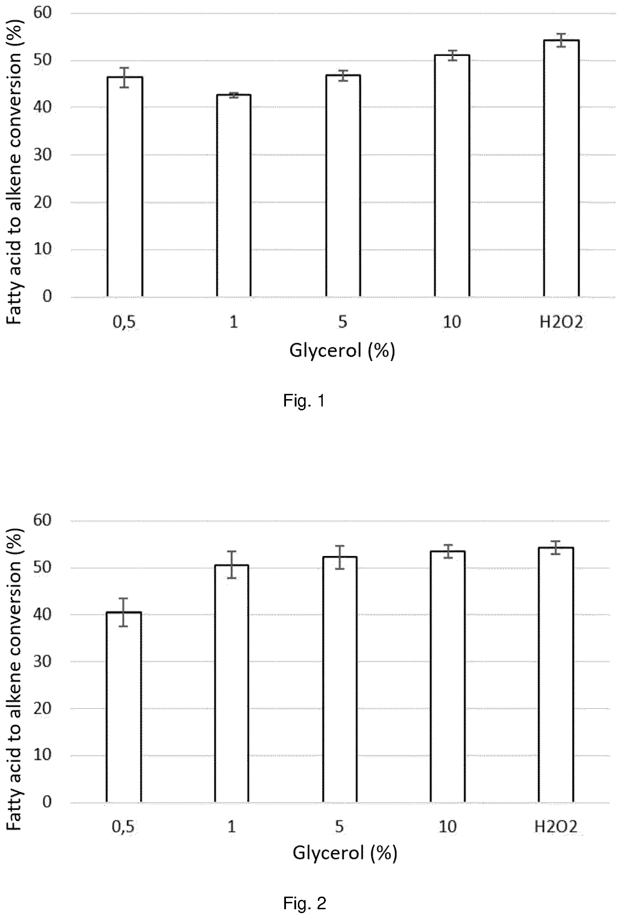

Figure 1 graphically presents the percentage of conversion of myristic acid (C14:0) to its corresponding terminal alkene (1-tridecene) by means of the chimeric enzyme of the present invention, using said enzyme with flexible linking sequence (SEQ ID NO: 3) at 1 µM, 0.5 mM myristic acid and concentrations between 0.5 and 10% of glycerol in the reaction medium (50 mM sodium phosphate buffer, pH 7.5). The reaction was carried out at 37 °C with slow stirring (300 rpm) for 30 min. Comparatively, the result of the conversion obtained using the decarboxylase enzyme (SEQ ID NO: 1) in the presence of hydrogen peroxide added to the medium (1 mM) is presented.
Figure 2 graphically presents the percentage of conversion of myristic acid (C14:0) to its corresponding terminal alkene (1-tridecene) by means of the chimeric enzyme of the present invention, using said enzyme with a rigid binding sequence (SEQ ID NO: 4) at 1 µM, 0.5 mM myristic acid and concentrations between 0.5 and 10% of glycerol in the reaction medium (50 mM sodium phosphate buffer, pH 7.5). The reaction was carried out at 37 °C with slow stirring (300 rpm) for 30 min. Comparatively, the result of the conversion obtained using the decarboxylase enzyme (SEQ ID NO: 1) in the presence of hydrogen peroxide added to the medium (1 mM) is presented.

### DETAILED DESCRIPTION OF THE INVENTION

As previously stated, the present invention concerns a chimeric enzyme constructed for the production of terminal alkenes from fatty acids, as well as the process for the production of said alkenes using said enzyme. From this point onwards, aspects of the present invention are detailed.

The chimeric enzyme for the production of terminal alkenes of the present invention is characterized, firstly, by the fact that it performs the conversion of fatty acids using glycerol as a substrate for the *in situ* production of hydrogen peroxide.

The chimeric enzyme for the production of terminal alkenes of the present invention is characterized by comprising the decarboxylase enzyme SEQ ID NO: 1 linked in its terminal portion to the beginning of the alditol oxidase enzyme SEQ ID NO: 2, hydrogen peroxide generating enzyme, by means of a linker sequence. Said binding sequence is preferably selected from the group comprising SEQ ID NO: 3 and SEQ ID NO: 4.

Thus, an object of the present invention is a chimeric enzyme for the production of terminal alkenes characterized by having SEQ ID NO: 5, which is the decarboxylase enzyme SEQ ID NO: 1, linked in its terminal portion to the SEQ ID binding sequence NO: 3, in turn linked to the initial portion of the alditol oxidase enzyme SEQ ID NO: 2.

Also an object of the present invention is a chimeric enzyme for the production of terminal alkenes characterized by having SEQ ID NO: 6, which is the decarboxylase enzyme SEQ ID NO: 1, linked in its terminal portion to the binding sequence SEQ ID NO: 4, in turn linked to the initial portion of the enzyme alditol oxidase SEQ ID NO: 2.

The chimeric enzyme of the present invention has application in the conversion of fatty acids to alkenes. Therefore, a process for producing terminal alkenes is also an object of the present invention.

The process of the present invention is characterized by comprising the steps of: a) Contacting chimeric enzyme with a fatty acid in the presence of glycerol, forming a reaction medium; b) Keeping the reaction medium under heating and stirring for a period of time; and, c) Collecting the product.

In step a), the chimeric enzyme to be used is the decarboxylase SEQ ID NO: 1 joined in its terminal portion to the beginning of the alditol oxidase SEQ ID NO: 2 by means of a linker sequence. Said binding sequence is preferably selected from the group comprising SEQ ID NO: 3 and SEQ ID NO: 4. More specifically, the chimeric enzyme to be employed in step a) can be selected from the group comprising SEQ ID NO: 5 and SEQ ID NO: 6.

The fatty acid may be selected from the group comprising capric acid (C10:0), lauric acid (C12:0), myristic acid (C14:0), palmitic acid (C16:0), stearic acid (C18:0), oleic acid (C18:1) or combinations thereof.

Furthermore, the preferred ratio of chimeric enzyme to fatty acid in the medium is 1.0 µMol L⁻¹ of enzyme for 0.5 mMol L⁻¹ of fatty acid. Glycerol can be in concentrations preferably between 0.5 and 10% in the reaction medium.

In step b), the reaction medium must be heated to a temperature between 30 and 40 °C, preferably 37 °C.

The agitation imposed on the reaction medium at this stage of the process must be mild, up to 500 rpm, preferably 300 rpm.

The pH of the reaction medium must be maintained between 7.0 and 8.0, preferably 7.5.

Finally, the reaction medium must be maintained under the conditions set out for, preferably, 30 minutes.

In step c), the collected product can optionally be purified. For such a step, any separation and purification techniques known to one skilled in the art may be employed.

Below, as a basis, but without being restricted to them, examples of embodiments of the present invention are presented.

### EXAMPLE 1: Chimeric enzyme expression procedure

The coding regions of the enzymes of the present invention (SEQ ID NO: 7 and SEQ ID NO: 8) were cloned individually into pET28a expression vectors, with a 6xHisTag tail to facilitate product purification. The recombinant vectors were transformed into *E*. *coli* BL21, and the expression of each of the chimeric enzymes was conducted in "Terrific Broth" (TB) medium. For expression, transformed bacteria were grown in 1L of TB medium, at 30 °C, containing the appropriate antibiotics. Upon reaching an optical density value equal to 1, the medium temperature was reduced to 18 °C and the induction of expression of the enzymes of the present invention was initiated with 0.2 mMol L⁻¹ de IPTG, 20 µMol L⁻¹ of hemin in 100 µMol L⁻¹ of 5-aminolevulinate. After 24h of induction, the cells were isolated and used to purify the chimeric enzymes. Purification was carried out on nickel resin, with 25 mMol L⁻¹ sodium phosphate buffer (pH 7.5) and 500 mMol L⁻¹ NaCl. Chimeric enzymes were recovered on an imidazole gradient, from 0-500 mMol L^{- 1}. Both constructions resulted in the production of soluble and functional chimeric enzymes (SEQ ID NO: 5 and SEQ ID NO: 6).

### EXAMPLE 2: Terminal alkene production assay with chimeric enzymes

Conversion reactions of myristic acid to its corresponding terminal alkene (1-tridecene) were carried out with the chimeric enzymes separately, using a final volume of 1 mL. In all cases, the reaction medium was composed of the chimeric enzyme to be tested, at a concentration of 1 µMol L⁻¹, the fatty acid, at a concentration of 0.5 mMol L⁻¹, and 50 mM sodium phosphate buffer, pH 7.5. Different concentrations of glycerol (0.5, 1, 5 or 10%) were added to each reaction to evaluate the conversion rate (%) of myristic acid into 1-tridecene. All reactions were carried out at 37 °C, with gentle stirring (300 rpm), for 30 min.

For comparison, a reaction with the traditional process of converting fatty acids to alkenes was carried out using the same process conditions, but replacing the chimeric enzymes with decarboxylase SEQ ID NO: 1 and glycerol with hydrogen peroxide at the concentration of 1mM (final).

As a result, the traditional condition allowed a conversion of myristic acid to 1-tridecene of around 55% to be achieved. As can be seen in Figure 1, the tests carried out with the chimeric enzyme SEQ ID NO: 5 of the present invention showed a conversion percentage between 40 and 50%, with the highest result being achieved with a concentration of 10% of glycerol. In Figure 2, where the performance results of the chimeric enzyme SEQ ID NO: 6 are presented, a conversion between 40 and 55% can be seen, reaching these levels from 5% of glycerol in the reaction medium.

In this way, it is observed that the present invention makes it possible to generate peroxide in the reaction medium from glycerol. Instead of adding hydrogen peroxide to the medium, the addition of glycerol brings a new perspective to the disposal of this industrial waste in an effective way, using a single chimeric enzyme, without the need to use two enzyme systems. to carry out the conversion reaction.

## Claims

1. Chimeric enzyme for the production of terminal alkenes **characterized by** comprising the decarboxylase enzyme SEQ ID NO: 1 joined in its terminal portion to the beginning of the alditol oxidase enzyme SEQ ID NO: 2 by means of a linker sequence.

2. Chimeric enzyme according to claim 1, **characterized by** the fact that the binding sequence is selected from the group comprising SEQ ID NO: 3 and SEQ ID NO: 4.

3. Chimeric enzyme, according to claim 1, **characterized by** having SEQ ID NO: 5.

4. Chimeric enzyme, according to claim 1, **characterized by** having SEQ ID NO: 6.

5. Process for the production of terminal alkenes **characterized by** comprising the steps of:
a) Contacting chimeric enzyme with a fatty acid in the presence of glycerol, forming a reaction medium;
b) Keeping the reaction medium under heating and stirring for a period of time, and
c) Collecting the product.

6. Process according to claim 5, **characterized by** the fact that the chimeric enzyme is the decarboxylase SEQ ID NO: 1 linked in its terminal portion to the beginning of the alditol oxidase SEQ ID NO: 2 by means of a linker sequence.

7. Process according to claim 6, **characterized by** the fact that the binding sequence is selected from the group comprising SEQ ID NO: 3 and SEQ ID NO: 4.

8. Process according to claim 5, **characterized by** the fact that the enzyme is selected from the group comprising SEQ ID NO: 5 and SEQ ID NO: 6.

9. Process according to claim 5, **characterized by** the fact that the fatty acid is selected from the group comprising capric acid (C10:0), lauric acid (C12:0), myristic acid (C14: 0), palmitic acid (C16:0), stearic acid (C18:0), oleic acid (C18:1) or combinations thereof.

10. Process according to claim 5, **characterized by** the fact that the preferred ratio between chimeric enzyme and fatty acid in the medium is 1.0 µMol L⁻¹ of enzyme for 0.5 mMol L⁻¹ of fatty acid.

11. Process, according to claim 5, **characterized by** the fact that the concentration of glycerol in the medium is, preferably, between 0.5 and 10%.

12. Process, according to claim 5, **characterized by** the fact that the reaction medium must be heated to a temperature between 30 and 40 °C, preferably to 37 °C.

13. Process, according to claim 5, **characterized by** the fact that agitation imposed on the medium is up to 500 rpm, preferably 300 rpm.

14. Process according to claim 5, **characterized by** the fact that the pH of the reaction medium is maintained between 7.0 and 8.0, preferably 7.5.

15. Process, according to claim 5, **characterized by** the fact that the time is, preferably, 30 minutes.
